# EUROPÄISCHE PATENTSCHRIFT

(11) **EP 0 539 990 B1**
(45) Veröffentlichungstag und Bekanntmachung des Hinweises auf die Patenterteilung: **08.01.1997**
(21) Anmeldenummer: 92118516.1
(22) Anmeldetag: 29.10.1992
(51) Int. Cl.: C07C 13/40, C07C 5/29

(54) **Verfahren zur Herstellung von Camphen durch Umlagerung von alpha-Pinen**
Process for the preparation of camphene by isomerisation of alpha-pinene
Procédé pour la préparation de camphène par isomérisation de alpha-pinène

(30) Priorität: 01.11.1991 DE 4136041
(43) Veröffentlichungstag der Anmeldung: 05.05.1993
(73) Patentinhaber: HOECHST AKTIENGESELLSCHAFT, 65926 Frankfurt am Main (DE)
(72) Erfinder: Ritter, Eberhard, Dr., W-6082 Mörfelden (DE); Wisser, Thomas, Dr., W-6250 Limburg (DE); Riedel, Alfred, Dr., W-8901 Stadtbergen (DE); Gscheidmeier, Manfred, Dr., W-8901 Gablingen (DE); Maginot, Joachim, W-8900 Augsburg (DE)

(56) Entgegenhaltungen:
- DE-B- 1 568 949
- US-A- 2 551 795
- CHEMICAL ABSTRACTS, vol. 99, no. 1, 4. Juli 1983, Columbus, Ohio, US; abstract no. 5875t, Seite 538 ;

## Beschreibung

Die Erfindung bezieht sich auf ein verbessertes Verfahren zur Herstellung von Camphen durch Umlagerung von α-Pinen, wobei Tricyclen und Dipenten als Hauptnebenprodukte anfallen:

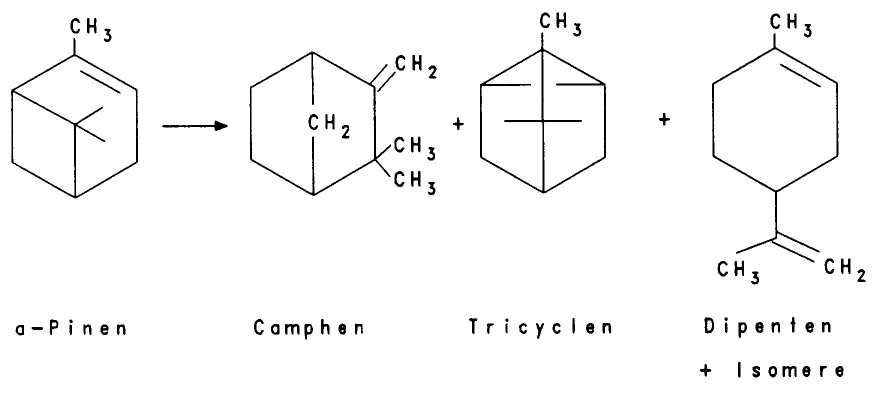

Camphen ist ein wichtiges Zwischenprodukt zur Herstellung von anderen Terpenderivaten, beispielsweise Isoborneol, Campher und Riechstoffen. Seit vielen Jahren wird die Umlagerung des α-Pinens zu Camphen (Wagner-Meerwein-Umlagerung) nach einem großtechnischen Verfahren durchgeführt, bei welchem die Reaktion in Form einer heterogenen Katalyse stattfindet (Katalysator: Titanoxid-hydrat), beispielsweise diskontinuierlich in Rührkesseln bei einer Reaktionstemperatur von ca. 130 °C.
Nach diesem Verfahren beansprucht die Umlagerung bei einer Temperatur von ca. 135 °C je nach Zusatz von frischem Katalysator 65 bis 85 Stunden.

Es ist bekannt, daß die Umlagerung von α-Pinen im Laboratoriumsmaßstab bei 160°C in Gegenwart eines TiO₂-Katalysators mit einer ca. 65%igen Ausbeute an Camphen möglich ist (vgl. C.A.,Vol. 89, 197 725g (1978)). Dabei beträgt die Reaktionszeit bei einer Katalysatorkonzentrationvon 1,5 bis 2,0 Gew.% etwa 160 min (=2,7 h).

Bekannt ist aber auch, daß im Laboratorium mit steigender Reaktionstemperatur die Ausbeute an Camphen sehr stark zurückgeht, wobei gleichzeitig die Konzentration an Polymeren stark ansteigt (vgl. C.A. Vol. 79, 5786 (1957)). Außerdem handelt es sich bei der Umlagerung des α-Pinens um eine stark exotherme Reaktion, die es bisher nicht erlaubte, in der Technik bei wesentlich größeren Ansätzen und schlechterem Wärmeübergang als im Labor bei höherer Temperatur zu arbeiten.

Weiterhin ist ein Verfahren bekannt zur Herstellung von Camphen durch Umlagerung von Pinen bei einem Unterdruck von ca. 6,6 bis 46,6 kPa (50 bis 350 mm) und einer Temperatur von etwa 80 bis 135°C in Gegenwart eines Katalysators aus amorphem hydratisierten Titandioxid, welches eine bestimmte Menge Wasser enthält (vgl. US-PS 2,551,795). Es wird darauf hingewiesen, daß Temperaturen oberhalb von 135°C die Ausbeute an Camphen beträchtlich verringern und daß bei der Rückflußtemperatur unter Normaldruck (155 bis 165°C) große Mengen anderer Kohlenwasserstoffe entstehen.

Schließlich ist ein Verfahren bekannt zur Herstellung von Camphen durch Isomerisierung von Pinen, bei welchem Titandioxid als Katalysator dient (vgl. (JP-A Sho 58-26826 (= C.A. Vol. 99, 5875 t)). Dieses Titandioxid wird gewonnen, indem man der Titansäure, die durch Hydrolyse einer Titanverbindung entseht, entweder Ammoniumsulfat oder Schwefelsäure zusetzt und das Ganze nach dem Entfernen von Wasser sintert. Der Katalysator wird als sehr aktiv und gut reproduzierbar beschrieben, er muß dennoch in relativ großer Menge eingesetzt werden.

Es bestand daher die Aufgabe, ein Verfahren zur Herstellung von Camphen zu finden, welches einerseits kürzere Reaktionszeiten bei gleichzeitig geringeren Mengen Katalysator ermöglicht und andererseits eine sichere Abfuhr der exothermen Energie gewährleistet.

Die Erfindung betrifft somit ein Verfahren zur Herstellung von Camphen durch Umlagerung von α-Pinen in Gegenwart eines Trtanoxidhydrat-Katalysators in der Wärme, dadurch gekennzeichnet, daß die Umlagerung in Gegenwart von 0,1 bis 2,0 Gew.-%, bezogen auf reines α-Pinen, des Katalysators bei einer Temperatur von 155 bis 165 °C unter Rückfluß des α-Pinens durchgeführt und die anfallende Reaktionswärme durch Siedekühlung abgeführt wird.

Das erfindungsgemäße Verfahren kann in den üblichen, in der chemischen Industrie verwendeten Rührbehältern durchgeführt werden. Zweckmäßigerweise werden Standard-Rührkessel mit aufgesetzten Kondensatoren eingesetzt. Möglich ist auch die Verwendung von Durchflußreaktoren, sofern sie gestatten, die Reaktionswärme effektiv abzuführen.

Das Verfahren wird in der Weise durchgeführt, daß das α-Pinen und der Katalysator bei diskontinuierlicher Arbeitsweise in den Rührkessel eingefüllt und mit einer Aufheizgeschwindigkeit von 0,5 bis 10, vorzugsweise 2 bis 4 °C/min auf Rückflußtemperatur (ca. 155 bis 165 °C) erhitzt werden. Dabei wird die Reaktionswärme durch Siedekühlung abgeführt. Die Reaktionszeit beträgt 0,3 bis 3,5, vorzugsweise 0,7 bis 1,5 h je nach Katalysatorkonzentration und Aufheizgeschwindigkeit. Das Reaktionsende macht sich durch einen starken Abfall der exothermen Reaktionsenergie bei gleichzeitig steigendem Siedepunkt des Produktes bemerkbar.

Der Katalysator ist ein Titanoxidhydrat, welches folgendermaßen gewonnen werden kann: Eine aus dem Ilmenit-Aufschluß stammende schwefelsaure Paste wird mit NaOH-Lösung versetzt, mehrere Stunden gerührt, gewaschen, mit Essigsäure abermals gerührt, abgenutscht und anschließend bei 50 bis 100, vorzugsweise bei 60 bis 80 °C im Vakuum getrocknet.

Die einzusetzende Menge an Katalysator beträgt 0,1 bis 2,0, vorzugsweise 0,3 bis 0,5 Gew.-%, bezogen auf reines α-Pinen.

Beim erfindungsgemäßen Verfahren verändern sich gegen Reaktionsende die Konzentrationen der wichtigsten Komponenten sehr deutlich, was für die Ausbeute, die Zusammensetzung des Reaktionsgemisches und den Reaktionsendpunkt von erheblicher Bedeutung ist. Durch genaue Temperatursteuerung (insbesondere gegen Ende der Reaktion) lassen sich Ausbeute, Zusammensetzung des Produktgemisches und Reaktionsendpunkt genau festlegen. So kann beispielsweise über die Abkühlgeschwindigkeit nach dem Abfall der exothermen Reaktionsenergie zum Beispiel das Verhältnis Camphen zu Tricyclen beeinflußt werden.

Die nachfolgenden Beispiele sollen die Erfindung verdeutlichen.

### Vergleichsbeispiel A

In einen 1l-Dreihalskolben (Glas) mit Rührer, Thermometer und Rückflußkühler wurden 300 g α-Pinen zusammen mit 1,5 g Katalysator (0,5 Gew.-%) auf 136 °C erwärmt (4 °C/min) und 20 h bei dieser Temperatur gerührt. Nach dieser Zeit war die Reaktion beendet und der Ansatz wurde rasch (10 °C/min) auf Raumtemperatur gekühlt. Die GC-Analyse des gefilterten Produktes ergab einen Camphen /Tricyclengehalt von 71,1 Gew.-%.

### Vergleichsbeispiel B

In einen 1l-Dreihalskolben (Glas) mit Rührer, Thermometer und Rückflußkühler wurden 300 g α-Pinen zusammen mit 1,5 g Katalysator (0,5 Gew.-%) auf 146 °C erwärmt (4 °C/min) und 5 h bei dieser Temperatur gerührt. Nach dieser Zeit war die Reaktion beendet und der Ansatz wurde rasch (10 °C/min) auf Raumtemperatur gekühlt. Die GC-Analyse des gefilterten Produktes ergab einen Camphen /Tricyclengehalt von 68,6 Gew.-%.

### Beispiel 1

In einen 1l-Dreihalskolben (Glas) mit Rührer, Thermometer und Rückflußkühler wurden 300 g α-Pinen zusammen mit 0,9 g Katalysator (0,3 Gew.-%) auf Siedetemperatur (155 bis 165 °C) erwärmt (4 °C/min) und 3,5 h bei dieser Temperatur gerührt. Nach dieser Zeit war die Reaktion beendet und der Ansatz wurde rasch (10 °C/min) auf Raumtemperatur gekühlt. Die GC-Analyse des gefilterten Produktes ergab einen Camphen /Tricyclengehalt von 69,2 Gew.-%.

### Beispiel 2

In einen 1l-Dreihalskolben (Glas) mit Rührer, Thermometer und Rückflußkühler wurden 300 g α-Pinen zusammen mit 1,5 g Katalysator (0,5 Gew.-%) auf Siedetemperatur (155 bis 165 °C) erwärmt (4 °C/min) und 1,5 h bei dieser Temperatur gerührt. Nach dieser Zeit war ein starker Abfall der exothermen Reaktionsenergie zu bemerken und das Gemisch wurde rasch (10 °C/min) auf Raumtemperatur gekühlt. Die GC-Analyse des gefilterten Produktes ergab einen Camphen /Tricyclengehalt von 68,8 Gew.-%. Das Verhältnis Camphen zu Tricyclen betrug 7,4 : 1.

### Beispiel 3

In einen 1l-Dreihalskolben (Glas) mit Rührer, Thermometer und Rückflußkühler wurden 300 g α-Pinen zusammen mit 1,5 g Katalysator (0,5 Gew.-%) auf Siedetemperatur (155 bis 165 °C) erwärmt (4 °C/min) und 1,5 h bei dieser Temperatur gerührt. Der dann auftretende Abfall der exothermen Reaktionsenergie wurde durch weiteres Erhitzen kompensiert und der Ansatz nochmals 10 min bei Siedetemperatur gerührt. Danach wurde er rasch (10 °C/min) auf Raumtemperatur gekühlt. Die GC-Analyse des gefilterten Produktes ergab einen Camphen /Tricyclengehalt von 69,1 Gew.-%. Das Verhältnis Camphen zu Tricyclen betrug 5,3 : 1.

### Beispiel 4

In einen 1l-Dreihalskolben (Glas) mit Rührer, Thermometer und Rückflußkühler wurden 300 g α-Pinen zusammen mit 1,5 g Katalysator (0,5 Gew.-%) auf Siedetemperatur (155 bis 165 °C) erwärmt (4 °C/min) und 1,5 h bei dieser Temperatur gerührt. Der dann auftretende Abfall der exothermen Reaktionsenergie wurde durch weiteres Erhitzen kompensiert und das Gemisch nochmals 20 min bei Siedetemperatur gerührt. Danach wurde es rasch (10 °C/min) auf Raumtemperatur gekühlt. Die GC-Analyse des gefilterten Produktes ergab einen Camphen /Tricyclengehalt von 69,0 Gew.-%. Das Verhältnis Camphen zu Tricyclen betrug 4,7 : 1.

### Beispiel 5

In einen 1l-Dreihalskolben (Glas) mit Rührer, Thermometer und Rückflußkühler wurden 300 g α-Pinen zusammen mit 3,0 g Katalysator (1,0 Gew.-%) auf Siedetemperatur (155 bis 165 °C) erwärmt (4 °C/min) und 0,7 h bei dieser Temperatur gerührt. Nach dieser Zeit war die Reaktion beendet und der Ansatz wurde rasch (10 °C/min) auf Raumtemperatur gekühlt. Die GC-Analyse des gefilterten Produktes ergab einen Camphen /Tricyclengehalt von 68,3 Gew.-%.

### Beispiel 6

In einem 400 l-Standard-Rührkessel (Metall) mit Rührer, Temperaturmessung, Kondensator und beheizbarem Doppelmantel (Wasserdampf 3 bzw. 6 bar) wurden 250 kg α-Pinen mit 1,25 kg Katalysator (0,5 Gew.-%) auf Siedetemperatur (155 bis 165 °C) erwärmt (3 °C/min) und 1,5 h bei dieser Temperatur gerührt. Nach dieser Zeit war ein deutlicher Abfall der exothermen Reaktionswärme bemerkbar und das Reaktionsgemisch wurde auf Raumtemperatur abgekühlt. Die GC-Analyse des gefilterten Produktes ergab einen Camphen/Tricyclengehalt von 69,0 Gew.-%. Das Verhältnis Camphen zu Tricyclen betrug 7,5 : 1.

## Patentansprüche

1. Verfahren zur Herstellung von Camphen durch Umlagerung von α-Pinen in Gegenwart eines Titanoxidhydrat-Katalysators in der Wärme, dadurch gekennzeichnet, daß die Umlagerung in Gegenwart von 0,1 bis 2,0 Gew.-%, bezogen auf reines α-Pinen, des Katalysators bei einer Temperatur von 155 bis 165°C unter Rückfluß des α-Pinens durchgeführt und die anfallende Reaktionswärme durch Siedekühlung abgeführt wird, wobei der Katalysator in der Weise hergestellt wird, daß eine aus einem Ilmenit-Aufschluß stammende schwefelsaure Paste mit einer NaOH-Lösung versetzt, mehrere Stunden gerührt, gewaschen, mit Essigsäure gerührt, abgenutscht und anschließend bei 50 bis 100, vorzugsweise bei 60 bis 80°C im Vakuum getrocknet wird.

2. Verfahren nach Anspruch 1, dadurch gekennzeichnet, daß der Reaktionsansatz mit einer Aufheizgeschwindigkeit von 0,5 bis 10 °C/min erwärmt wird.

## Claims

1. A process for the preparation of camphene by the rearrangement of α-pinene, in the presence of a titanium oxide hydrate catalyst, under the action of heat, which comprises carrying out the rearrangement in the presence of from 0.1 to 2.0% by weight, based on pure α-pinene, of a catalyst at a temperature of 155 to 165°C under reflux of the α-pinene and eliminating the heat of reaction produced by evaporative cooling, the catalyst being prepared in such a manner that a paste acidified with sulfuric acid, originating from a digestion of ilmenite, is treated with an NaOH solution, the mixture is stirred for several hours, washed and stirred with acetic acid and the product is filtered off with suction and then dried under vacuum at 50 to 100, preferably at 60 to 80°C.

2. The process as claimed in claim 1, wherein the reaction mixture is heated at a rate of 0.5 to 10°C/min.

## Revendications

1. Procédé pour préparer le camphène par transposition de l'α-pinène en présence d'un catalyseur à base d'oxyde de titane hydraté, à chaud, caractérisé en ce que la transposition est mise en oeuvre en présence de 0,1 à 2,0 % en poids, par rapport à l'α-pinène pure, du catalyseur à une température de 155 à 165°C, au reflux de l'α-pinène, et la chaleur de réaction qui se produit est évacuée par refroidissement par vaporisation, auquel cas on prépare le catalyseur en ajoutant une solution de NaOH à une pâte acidifiée à l'acide sulfurique, obtenue par attaque de l'ilménite, on l'agite pendant plusieurs heures, on la lave, on l'agite avec de l'acide acétique, on la filtre à la trompe, puis on la sèche sous vide à une température de 50 à 100 et de préférence de 60 à 80°C.

2. Procédé selon la revendication 1, caractérisé en ce que la masse réactionnelle est chauffée à une vitesse de montée en température de 0,5 à 10°C/minute.
